# EUROPEAN PATENT APPLICATION

(11) **EP 2 446 929 A1**
(43) Date of publication of application: **02.05.2012**
(21) Application number: 10189093.7
(22) Date of filing: 27.10.2010
(51) Int. Cl.: A61P 9/10, A61K 48/00, A61K 9/51

(54) **Microvesicles derived from atheroprotective endothelial cells for the treatment and prevention of atherosclerotic diseases**

(71) Applicant: Johann Wolfgang Goethe-Universität Frankfurt am Main, 60325 Frankfurt am Main (DE)
(72) Inventor: Boon, Reinier, 60596, Frankfurt (DE); Dimmeler, Stefanie, 60594, Frankfurt (DE); Hergenreider, Eduard, 60327, Frankfurt am Main (DE)
(74) Representative: Krauss, Jan

(57) **Abstract**

The present invention relates to methods for the production of atheroprotective vesicles. The invention is based on the finding that endothelial cells wherein the activity of the transcription factor KLF2 is induced and/or enhanced surprisingly produce and/or secrete vesicles enclosing various atheroprotective microRNAs, in particular microRNAs of the microRNA-cluster miR-143/145. Thus, the present invention discloses methods for the production of vesicles derived from atheroprotective endothelial cells and the vesicles produced by the disclosed methods. Furthermore artificially produced vesicles enclosing the atheroprotective microRNA composition according to the invention are provided. The vesicles of the present invention are useful in the treatment and prevention of atherosclerotic diseases, such as acute coronary syndromes, plaque rupture, aneurysms, in-stent restenosis, ischemic diseases and heart failure.

## Description

### Field of the Invention

The present invention relates to methods for the production of atheroprotective vesicles. The invention is based on the finding that endothelial cells wherein the activity of the transcription factor KLF2 is induced and/or enhanced surprisingly produce and/or secrete vesicles enclosing various atheroprotective microRNAs, in particular microRNAs of the microRNA-cluster miR-143/145. Thus, the present invention discloses methods for the production of vesicles derived from atheroprotective endothelial cells and the vesicles produced by the disclosed methods. Furthermore artificially produced vesicles enclosing the atheroprotective microRNA composition according to the invention are provided. The vesicles of the present invention are useful in the treatment and prevention of atherosclerotic diseases, such as acute coronary syndromes, plaque rupture, aneurysms, in-stent restenosis, ischemic diseases and heart failure.

### Background of the Invention

Atherosclerosis and its complications are still the main cause of death in the western world and due to the increase in life expectancy and the increased age of the overall population, age-associated diseases like atherosclerosis are expected to further increase over time. Therefore, an efficient treatment is desperately needed. Current attempts to therapeutically interfere with abnormal vessel remodeling and plaque rupture focus on taming the inflammatory response associated with alterations of vascular wall structures. However, there are limited therapeutic options to modify smooth muscle cell functions during plaque destabilization.

Atherosclerosis, the underlying cause of myocardial infarction and stroke, occurs only in predisposed spots in the large arteries, despite the systemic nature of the classical risk factors for atherosclerosis. The focal nature of atherosclerosis is the result of difference in local blood flow patterns across the endothelium (Davies, P.F., Polacek, D.C., Handen, J.S., Helmke, B.P. and DePaola, N. (1999) A spatial approach to transcriptional profiling: mechanotransduction and the focal origin of atherosclerosis. Trends Biotechnol, 17, 347 - 351). Endothelial cells that are exposed to laminar blood flow experience high shear stress and are protected from atherosclerosis formation, whereas turbulent blood flow, usually near bends and bifurcations, generates low endothelial shear stress, which leads to atherosclerosis formation.

In recent years it has become clear that the transcription factor Krüppel-like factor 2 (KLF2) plays a central role in mediating the atheroprotective endothelial phenotype generated by shear stress (Boon, R.A. and Horrevoets, A.J. (2009) Key transcriptional regulators of the vasoprotective effects of shear stress. Hamostaseologie, 29, 39 - 40, 41 - 33). The general KLF2-/- mouse dies at embryonic day 10.5, due to lack of vascular tone, bleeding and cardiac dysfunction secondary to the vascular complications (Kuo CT, Veselits ML, Barton KP, Lu MM, Clendenin C, Leiden JM. The LKLF transcription factor is required for normal tunica media formation and blood vessel stabilization during murine embryogenesis. Genes Dev. 1997 Nov 15;11(22):2996-3006). Interestingly enough, even the endothelial-restricted KLF2-/- exhibits apparently normal endothelium, but dysfunctional and dysorganized smooth muscle cells (SMCs).

One possible explanation for this phenomenon is that KLF2 normally directs communication between endothelial cells and SMCs, which is hampered in the KLF2-/- mouse. Communication between cells can be mediated by microvesicles, small right-side-out phospholipid bilayer encompassed vesicles containing protein, nucleic acids and other cellular components (Camussi, G., Deregibus, M.C., Bruno, S., Cantaluppi, V. and Biancone, L. (2010) Exosomes/microvesicles as a mechanism of cell - to - cell communication. Kidney Int). Apoptotic bodies derived from endothelial cells have recently been shown to induce atheroprotection in a mouse model (Zernecke, A., Bidzhekov, K., Noels, H., Shagdarsuren, E., Gan, L., Denecke, B., Hristov, M., Koppel, T., Jahantigh, M.N., Lutgens, E. et al. (2009) Delivery of microRNA - 126 by apoptotic bodies induces CXCL12 - dependent vascular protection. Sci Signal, 2, ra81). In that study the atheroprotective properties were attributed to the transfer of a microRNA, miR-126, via the apoptotic bodies.

MicroRNAs (miRNAs) are Small noncoding RNAs regulating gene expression on the posttranscriptional level by degradation of the target mRNA or translational repression. In contrast to small interference RNAs (siRNA), miRNA show less target specificity and bind to various mRNAs - preferably to their 3 prime untranslated regions. The regulation of a set of genes (in contrast to monotherapy by one gene or growth factor) does provide a distinctive advantage when interfering with complex processes like atherosclerosis. More than 1000 miRNAs have been identified so far in the human genome, but the relevance of most of these microRNAs for cellular function in physiological and pathological processes is unclear.

MicroRNAs are processed by the enzymes Drosha and Dicer, and incorporated in RNA-induced silencing complexes, that mediate the translational inhibition or degradation of target mRNAs (Bartel, D.P. (2004) MicroRNAs: genomics, biogenesis, mechanism, and function. Cell, 116, 281 - 297; Bartel, D.P. (2009) MicroRNAs: target recognition and regulatory functions. Cell, 136, 215 - 233.). Many miRNAs have been identified to play key roles in (patho)physiological processes, including atherosclerosis (Montgomery, R.L. and van Rooij, E. (2010) MicroRNA regulation as a therapeutic strategy for cardiovascular disease. Curr Drug Targets, 11, 936 - 942). One cluster of miRNAs, comprising miR-143 and miR-145 (miR-143/145), has been described to be of crucial importance for proper SMC function (Cordes, K.R., Sheehy, N.T., White, M.P., Berry, E.C., Morton, S.U., Muth, A.N., Lee, T.H., Miano, J.M., Ivey, K.N. and Srivastava, D. (2009) miR - 145 and miR - 143 regulate smooth muscle cell fate and plasticity. Nature, 460, 705 - U780; Elia, L., Quintavalle, M., Zhang, J., Contu, R., Cossu, L., Latronico, M.V., Peterson, K.L., Indolfi, C., Catalucci, D., Chen, J. et al. (2009) The knockout of miR - 143 and - 145 alters smooth muscle cell maintenance and vascular homeostasis in mice: correlates with human disease. Cell Death Differ, 16, 1590 - 1598;. Boettger, T., Beetz, N., Kostin, S., Schneider, J., Kruger, M., Hein, L. and Braun, T. (2009) Acquisition of the contractile phenotype by murine arterial smooth muscle cells depends on the Mir143/145 gene cluster. J Clin Invest, 119, 2634 - 2647).

The disclosure of WO 2009/105759 describes the identification of SMC regulating microRNAs in the context of cell proliferation and differentiation. Amongst others, the WO further describes members of the miR-143/miR-145 cluster. Two microRNAs were identified, miR-486 and miR-422a, that regulate cell survival in the heart. Thus WO 2009/105759 teaches methods of treating or preventing cardiac hypertrophy, heart failure, or myocardial infarction by increasing expression of miR-486 and/or miR-422a in heart tissue.

WO 2010/104796 provides genetically modified cells comprising exogenous miR-143 and/or miR-145 nucleic acids; and artificial tissues comprising the genetically modified cells that could be of use in methods and compositions for reducing pathological angiogenesis and for inhibiting vascular smooth muscle cell proliferation

MicroRNAs (miRNAs) are a class of noncoding RNAs that regulate target gene expression at the posttranscriptional level. It was reported that secreted miRNAs can serve as signaling molecules mediating intercellular communication. In human blood cells and cultured THP-1 cells, miR-150 was found to be selectively packaged into microvesicles (MVs) and actively secreted. The THP-1-derived MVs can enter and deliver miR-150 into human HMEC-1 cells, and elevated exogenous miR-150 effectively reduced c-Myb expression and enhanced cell migration in HMEC-1 cells (Zhang Y, Liu D, Chen X, Li J, Li L, Bian Z, Sun F, Lu J, Yin Y, Cai X, Sun Q, Wang K, Ba Y, Wang Q, Wang D, Yang J, Liu P, Xu T, Yan Q, Zhang J, Zen K, Zhang CY, Secreted monocytic miR-150 enhances targeted endothelial cell migration. Mol Cell. 2010 Jul 9;39(1):133-44:).

Although K1f2 is known to provide atheroprotective effects, this knowledge was not exploitable for therapeutic purposes. Biologically generated microparticles or vesicles were shown to deliver genes or microRNAs, however, so far no study addresses the generation of microparticles from specifically modified cells, which will express a defined pattern of atheroprotective microRNAs. Thus, while anti-atherosclerotic treatment strategies focused on a single gene or growth factor, the use of vesicles which are enriched in a set of microRNAs likely will have profound influences to the vessel wall. Since K1f2 improves both, the endothelial cell function, and the smooth muscle cells, the microvesicles generated by KIf2-transduced cells are predicted to provide atheroprotective effects on several levels.

### Summary of the Invention

In view of the state of the art it was an object of the present invention to provide novel therapeutic means for the treatment and prevention of diseases which root in atherosclerotic defects. Specifically, the present invention seeks to solve the problem of providing methods and means for a therapeutic treatment of atherosclerotic diseases based on the atheroprotective effects of the transcriptional regulator K1f2 - a knowledge which although being a promising starting point could not be transferred into a therapy in the clinic thus far.

The above problem is solved in a first aspect by a method for the production of atheroprotective vesicles, comprising the steps of
a. providing (cultured) endothelial cells,
b. inducing and/or enhancing the activity of the Krüppel-like factor 2 (Klf2) in said endothelial cells,
c. incubating said endothelial cells for a time sufficient to allow the cells to produce vesicles, and
d. optionally, isolating said vesicles.

In context of the present invention the term "vesicle" shall be understood as a supramolecular assembly made up of many different molecules. More technically, a vesicle is a small membrane-enclosed bubble that can store or transport substances - preferably for the present invention a vesicle encloses, amongst other molecules, microRNAs. Vesicles can form naturally because of the properties of lipid membranes, or they may be artificially prepared, for example in the form of micro or nano particles. For the present disclosure the term "vesicle" shall encompass all spherical structures of different sizes including micro vesicles composed of one or more layers of amphiphilic molecules (surfactants).

Specifically, "vesicles" may comprise both micelles, which have only one layer of amphiphilic molecules and are an aggregate of surfactant molecules dispersed in a liquid colloid, and liposomal structures. A typical micelle in aqueous solution forms an aggregate with the hydrophilic "head" regions in contact with surrounding solvent, sequestering the hydrophobic single tail regions in the micelle centre. Vesicles that are composed of at least one bilayer of surfactant molecules are liposomal vesicles. Liposomes can be composed of naturally-derived phospholipids with mixed lipid chains (like phosphatidylethanolamine) or other surfactants. If there is only one phospholipid bilayer, they are called unilamellar vesicles; otherwise they are called multilamellar.

Vesicles according to the present invention can be either naturally produced by a method according to the invention or artificially assembled, by adding the inventive atheroprotective microRNAs composition into a vesicular structure. Preferably, cell-derived vesicles are secreted vesicles and are preferably not formed via the process of cellular apoptosis - like for example apoptotic bodies. Means for an artificial assembly of vesicles are well known in the art.

Therefore, an "atheroprotective vesicle" shall for the purpose of the present invention relate to a vesicle that encloses at least one, preferably a composition, of the atheroprotective microRNAs disclosed by the present invention.

Further preferred is in one embodiment that the herein described methods for the production of atheroprotective vesicles are performed as *in-vitro* or *ex-vivo* methods. Thus meaning, that the methods of the present invention are not directly practised on an animal or human body. Thus, the term "providing endothelial cells" means that the cells are provided *in vitro* in a cultured cell system. Any method performed or practised directly on an animal or human body are not encompassed by the present invention. Endothelial cells usable for the method of the invention are preferably mammalian cells, more preferably human cells, in particular human vascular cells derived from an adult blood vessel or embryonic umbilical vein (HUVECs). All known primary endothelial cells or endothelial cell lines are preferred in the context of the present invention, specifically microvascular endothelial cells.

In a further preferred embodiment of the present invention, "inducing and/or enhancing the activity of KLF2" shall encompass any means that directly or indirectly improve the biological function of KLF2, in particular its function as a transcriptional regulator. Therefore, in the context of the present invention, the term "inducing and/or enhancing the activity of K1f2" comprises in one embodiment the introduction of a KLF2 expression construct into the endothelial cells and the subsequent expression of KLF2 molecules in said endothelial cells via said expression construct. By this way the number of active KLF2 protein molecules in the cell is elevated. This improves the activity of KLF2 as more KLF2 molecules may occupy the transcriptional promoter regions specific for KLF2 as compared to the situation in an untransduced cell. The person of skill in the relevant art is well acquainted with the transduction or transfection of recombinant genetic constructs and their expression in a target cell.

To this end it is encompassed by the present invention that the expression construct can be made of any construct known in the art, e.g. a viral, bacterial or plasmid based construct, so long as it is suitable for the introduction and expression of a KLF2 construct in the target cells. Preferred in the context of the invention are viral expression constructs, specifically a lentiviral expression system as e.g. described previously (Dekker, R.J., van Thienen, J.V., Rohlena, J., de Jager, S.C., Elderkamp, Y.W., Seppen, J., de Vries, C.J., Biessen, E.A., van Berkel, T.J., Pannekoek, H. et al. (2005) Endothelial KLF2 links local arterial shear stress levels to the expression of vascular tone - regulating genes. Am J Pathol, 167, 609 - 618).

Moreover, the term "KLF2" shall not only relate to the full length wild-type human KLF2 as used in the examples of the present invention, but shall encompass any functional equivalent molecules of KLF2. Functional equivalent molecules may comprise for example fragments of the full length protein which however still harbour the biological function and/or transcriptional activity of the wild type KLF2 protein. Also naturally occurring proteins similar to human KLF2, like paralougues, orthologues or known homologues shall be encompassed by the term "KLF2". Any protein which shares a high degree of homology and/or sequence identity to human KLF2 is a "KLF2" according to the present invention. Preferably the degree of sequence identity, either to the nucleic acid sequence or amino acid sequence of KLF2, is at least about 50%, 60%, 70%, 80%, 90%, 95% or preferably 98% compared to the human KLF2 nucleic acid and/or amino acid sequence under the proviso that the protein still harbours the function and/or activity of human KLF2.

In a further embodiment of the present invention "inducing and/or enhancing the activity of K1f2" can be achieved alternatively or additionally by applying shear stress to the endothelial cells. In the apparatuses and methods of this invention, shear stress can be applied to the cultured cells by various means. For example, shear stress can be applied by growing an endothelial cell monolayer on the surface of a rotating drum or rotating disc immersed in liquid growth medium. Alternatively, shear stress can be applied by growing an endothelial cell monolayer on static plates past which liquid growth medium is pumped. Shear stress can also be applied to a 3-dimensional endothelial cell culture by establishing the culture in a chamber through which liquid growth medium is pumped. Preferably, for the present invention a monolayer of endothelial cells, e.g. HUVECs, are exposed to a well-defined laminar flow. Preferably this laminar flow induces a shear flow stress of about >12 Dynes/cm², preferably about 20 Dynes/cm². The shear stress shall be applied for a time sufficient to activate KLF2 in the target cells. Preferably the shear stress is applied for 24 to 96 hours, most preferably for about 72 hours. In a preferred embodiment the flow is controlled by a perfusion system, preferably an Ibidi® perfusion system.

In a next embodiment of the present invention "inducing and/or enhancing the activity of K1f2" can be achieved alternatively or additionally by a treatment of said endothelial cells with one or more inducers of KLF2. The term "inducer of KLF2" refers to any molecule that exhibits either directly or indirectly an effect of facilitating the biological function of KLF2. Specifically, KLF2s function as a transcriptional regulator. In a preferred embodiment the inducer of Klf2 is a HMG-CoA reductase inhibitor, specifically selected from the group of the statins.

Statins (or HMG-CoA reductase inhibitors) are a class of drug used to lower cholesterol levels by inhibiting the enzyme HMG-CoA reductase, which plays a central role in the production of cholesterol in the liver. Increased cholesterol levels have been associated with cardiovascular diseases (CVD), and statins are therefore used in the prevention of these diseases. For the present invention preferably the statin is selected from the group consisting of Atorvastatin, Cerivastatin, Fluvastatin, Lovastatin, Mevastatin, Pitavastatin, Pravastatin, Rosuvastatin and Simvastatin, or combinations thereof. The most preferred statin is Mevastatin.

In one embodiment the endothelial cells are after the "inducing and/or enhancing the activity of Klf2" incubated for a time sufficient to allow the production and/or secretion of micro vesicles. Preferred is for the context of the present invention that the cells are cultivated for at least 24 hours, preferably for at least 48 hours, most preferably for at least 72 hours to allow the formation of the inventive atheroprotective vesicles.

In a screen for microRNAs that are regulated by KLF2, the inventors discovered that KLF2 surprisingly not only changes the microRNA pattern in endothelial cells, but its overexpression also leads to the secretion of microvesicles that are enriched with a unique pattern of microRNAs. Among the microRNAs that were shown to be enriched in microvesicles derived from KLF2-transduced cells, the atheroprotective microRNAs miR-143 and miR-145 were highly expressed. Both of these microRNAs were shown to be decreased in human atherosclerotic lesions and the regulated smooth muscle cell functions. Indeed, the inventors demonstrated that microvesicles isolated from Klf2-transduced cells modulate the gene expression pattern in smooth muscle cells. Since microvesicles can be systemically delivered and are efficiently taken up by atherosclerotic lesions, such atheroprotective microvesicles are thus useful to prevent or treat atherosclerosis.

Hence, it is a preferred embodiment of the invention that in a final step of the above method the vesicles are isolated from the endothelial cell culture. The isolation of said vesicles is to this end conducted starting from the endothelial cell culture, preferably from the culture supematant of endothelial cells. After the suitable cultivation as described herein above, the supernatant of the cell culture is in a preferred embodiment collected and the vesicles are isolated from said supernatant by means of a multi-step centrifugation. Method of isolating vesicles from a supernatant is well known in the art and encompassed by the present invention.

In a second aspect the problem of the present invention is solved by a vesicle produced with a method as described herein above. Also preferred is a vesicle that is obtainable by a method described by the present invention. Preferably, the vesicle is an isolated vesicle, recovered by the above described methods for isolation of vesicles.

A preferred vesicle according to the present invention enlcoses at least such miRNAs that show a KLF2 dependent expression in endothelial cells.

In a third aspect the problem is solved by a vesicle, enclosing at least one atheroprotective microRNA, preferably selected from the group consisting of miR-150, miR-451, miR-145, miR-143, miR-144, miR-193a-3p, miR-155, miR-181a, miR-214, miR-199b, miR-199a, miR-210, miR-146a, miR-126, miR-378, miR-363 and miR-30b. Most preferred for the present invention is a vesicle comprising at least the microRNAs miR-145 and miR-143.

In another embodiment of the present invention a vesicle is provided which encloses a composition of microRNAs comprising miR-145 and miR-143 and at least one further miRNA selected from the group of miR-451, miR-150, miR-144, miR-193a-3p, miR-155, miR-181a, miR-214 and miR-199a.

Yet another preferred vesicle is provided, wherein a microRNA composition in enclosed comprising at least the miRNAs miR-145, miR-143, miR-150, miR-144, miR-199a and/or miR-451.

In another embodiment, vesicles as described above are preferred wherein at least one, preferably both, of the miRNAs miR-126 and miR-210 are not present, or present in low concentrations or similar concentrations compared to vesicles derived from endothelial cells wherein KLF2 was not enhanced and/or induced in the meaning of the present invention.

In particular preferred is a vesicle as described above, wherein the micro RNAs are present in a concentration sufficient to alter gene expression in a target cell, preferably in a target smooth muscle cell.

For the present invention it is preferred that the inventive vesicle displays atheroprotective characteristics, specifically the vesicle according to the invention can be taken up by smooth muscle cells and change the gene expression of said smooth muscle cells. Specifically the inventive vesicles change the phenotype of said smooth muscle cells towards the "contractile" phenotype. Also preferred is an inventive vesicle that enhances angiogenesis, specifically endothelial cell angiogenesis.

Plaque rupture leading to acute ischemic events, like acute coronary syndromes (acute myocardial infarction and unstable angina) as well as ischemic stroke and acute peripheral artery occlusive disease, is due to thinning and break-down of the fibrous cap of an atheroma and ongoing dysfunction (change of contractile to synthetic phenotype) of vascular smooth muscle cells.

In a fourth aspect the problem of the invention is also solved by a vesicle as described herein for use in the treatment or prevention of a vascular disease. While anti-atherosclerotic treatment strategies focused on a single gene or growth factor, the use of microvesicles which are enriched in a set of microRNAs likely will have profound influences on the vessel wall. Since Klf2 improves both, the endothelial cell function, and the smooth muscle cells, the microvesicles generated by KIf2-transduced cells are predicted to provide atheroprotective effects on several levels. Since vesicles can be systemically delivered and are efficiently taken up by atherosclerotic lesions, such atheroprotective micro vesicles might be useful to prevent or treat atherosclerosis.

In one preferred embodiment of the invention the vascular disease is selected from the group consisting of acute coronary syndromes, such as myocardial infarction, unstable angina or sudden cardiac death, plaque rupture, aneurysms, in-stent restenosis and ischemic diseases, such as ischemic stroke, peripheral arterial occlusive disease, acute and chronic ischemic cardiac disease. Further preferred medical indications in context of the present invention are all types of heart failure, hibernating myocardium and microcirculory dysfunction.

A fifth aspect of the invention thus relates to a pharmaceutical composition, comprising a vesicle as described herein above, preferably formulated together with a pharmaceutical acceptable carrier and/or exipient. The pharmaceutical composition of the invention is preferably formulated for oral, buccal, parenteral, topical, and rectal or transdermal administration, or in a form suitable for administration by inhalation or insulation (either through the mouth or the nose).

For oral administration, the pharmaceutical compositions may take the form of, for example, tablets or capsules. The tablets may be coated by methods well known in the art. Liquid preparations for oral administration may take the form of, for example, solutions, syrups or suspensions or they may be presented as a dry product for constitution with water or other suitable vehicles before use. Such liquid preparations may be prepared by conventional means with pharmaceutically acceptable additives such as suspending agents; emulsifying agents; non-aqueous vehicles; and preservatives. The preparations may also contain buffer salts, flavouring, colouring and sweetening agents as appropriate.

Preparations for oral administration may be suitably formulated to give controlled release of the active compound. For buccal administration the compositions may take the form of tablets or lozenges formulated in a conventional manner.

The atheroprotective vesicles according to the present invention may be formulated for parenteral administration by injection, e.g. by bolus injection or continuous infusion. Formulations for injection may be presented in unit dosage form, e.g. in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g. sterile pyrogen-free water, before use.

In addition to the formulations described previously, the compositions of the invention may also be formulated as a depot preparation, e.g. in form of a biocompatible gel. Such long acting formulations may be administered by implantation (for example subcutaneously, transcutaneously or intravascular). The formulation can be injected into or near a treatment site. Thus, for example, the compounds according to the present invention may be formulated with suitable polymeric or hydrophobic materials (for example as emulsion in an acceptable oil) or ion exchange resins or as sparingly soluble derivatives, for example, as a sparingly soluble salt.

In some embodiments it may be desirable to deliver the active agent directly to the wall of a blood vessel. One exemplary method of vessel wall administration involves the use of a drug delivery catheter, particularly a drug delivery catheter comprising an inflatable balloon that can facilitate delivery to a vessel wall. In another example, the catheter is a perfusion balloon catheter, which allows perfusion of blood through the catheter while holding the formulation against the vessel walls for longer adsorption times.

A sixth aspect of the invention relates to a method of modulating a smooth muscle cell, comprising the steps of
a. providing a smooth muscle cell,
b. treating the smooth muscle cell with vesicles according to any one of claims 12 to 15, or
c. co-culturing said smooth muscle cell with endothelial cells wherein the activity of Klf2 is induced and/or enhanced; wherein the smooth muscle cell is preferably a mammalian cell, most preferably a human cell, such as a vascular smooth muscle cell, for example a human aortic smooth muscle cell.

A method for modulating is in one embodiment performed as an *in-vitro* or *ex-vivo* method. In one further embodiment the smooth muscle cell is provided in the context of a tissue, preferably wherein the smooth muscle cells are present in a blood vessel. Such a tissue or blood vessel may be an explanted tissue or blood vessel or an artificial tissue or blood vessel.

While the present invention has been described with specificity in accordance with certain of its preferred embodiments, the following examples serve only to illustrate the invention and are not intended to limit the invention within the principles and scope of the broadest interpretations and equivalent configurations thereof.

The invention is illustrated in the figures in which:

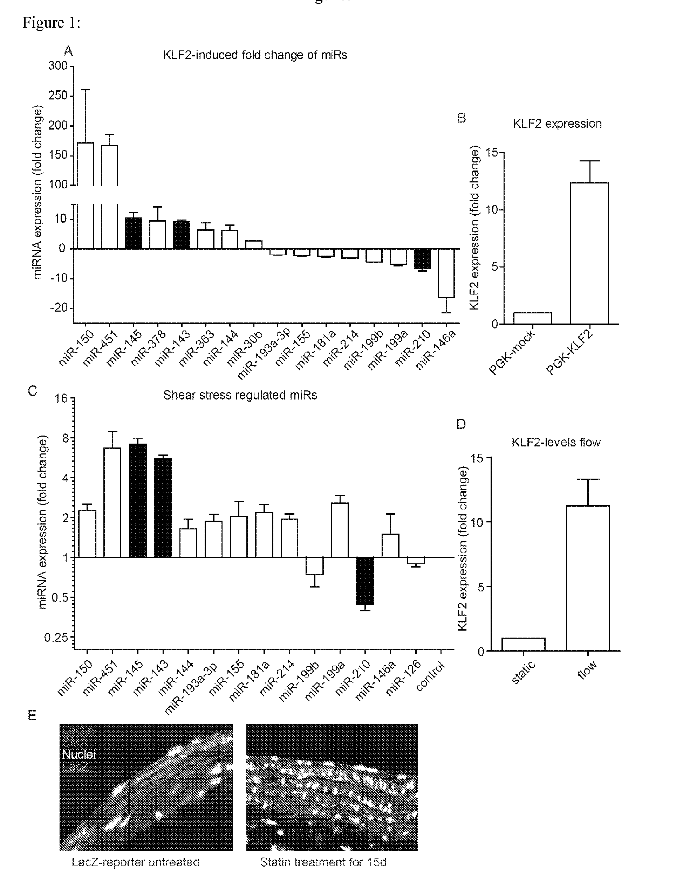

- **Figure 1**: shows KLF2 and shear stress induce endothelial expression of miR-143/145. HUVECs were transduced with KLF2-overexpression or mock-control lentivirus, RNA was isolated after 7 days and the indicated miRNAs and KLF2 expression levels were measured by real-time PCR (A and B). HUVECs were exposed to shear stress for 3 days and RNA isolation and real-time PCR were performed as in (A) and (B) (C and D). Aorta sections of LacZ-reporter mice for miR-143/145 were analyzed for LacZ expression by immunofluorescence (E). miR-143/145 expression is visualized in green, endothelial cells in red and nuclei in blue.

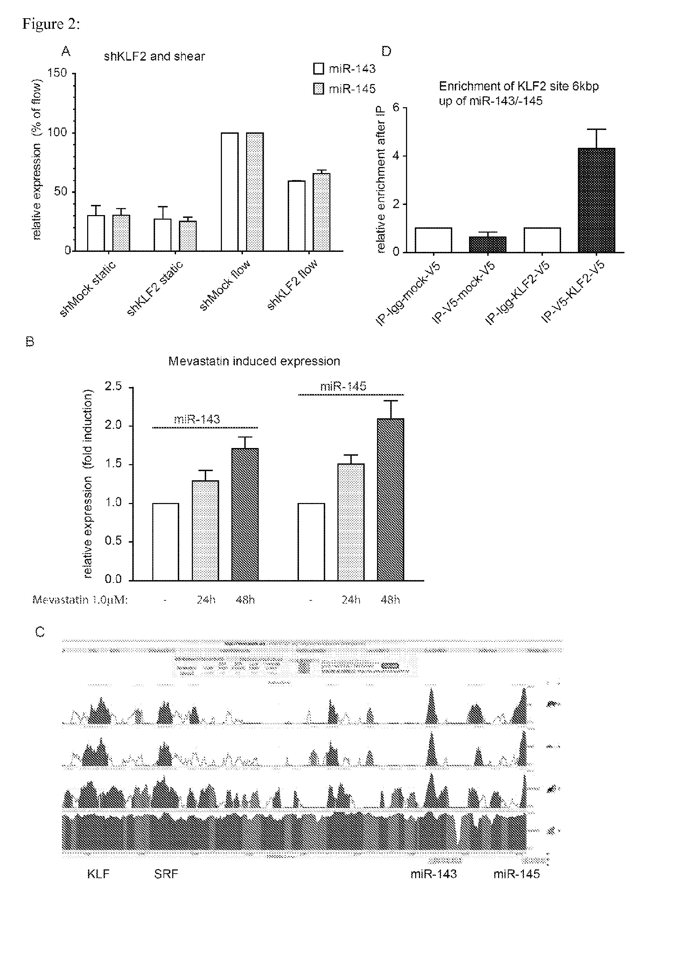

- **Figure 2**: shows shear stress and statins induce miR-143/145 expression through KLF2, which binds directly to the miR-143/145 promoter. HUVECs were transduced with a lentiviral shRNA construct to silence KLF2 expression or with a non-specific shRNA and exposed to laminar shear stress for 3 days or kept under static conditions (A). MiR-143 (white bars) and miR-145 (grey bars) expression was measured by real-time PCR. HUVECs were exposed to 1 µM Mevastatin for 24 (light grey bars) and 48 hours (dark grey bars) or left untreated (white bars) and miR-143 and miR-145 were measured by real-time PCR (B). Panel (C) illustrates the genomic region upstream of miR-143/145 and its evolutionary conservation. Examples of conserved transcription factor binding sites are indicated below the conserved regions. The base genome is human. HUVECs were transduced with lentivirus for V5-tagged KLF2 or control virus and immunoprecipitation was performed with V5 antibody (black bars) or unspecific IgG (white bars) (D). Real-time PCR was performed using primers specific for the region that contains the KLF binding site on chromatin that was precipitated.

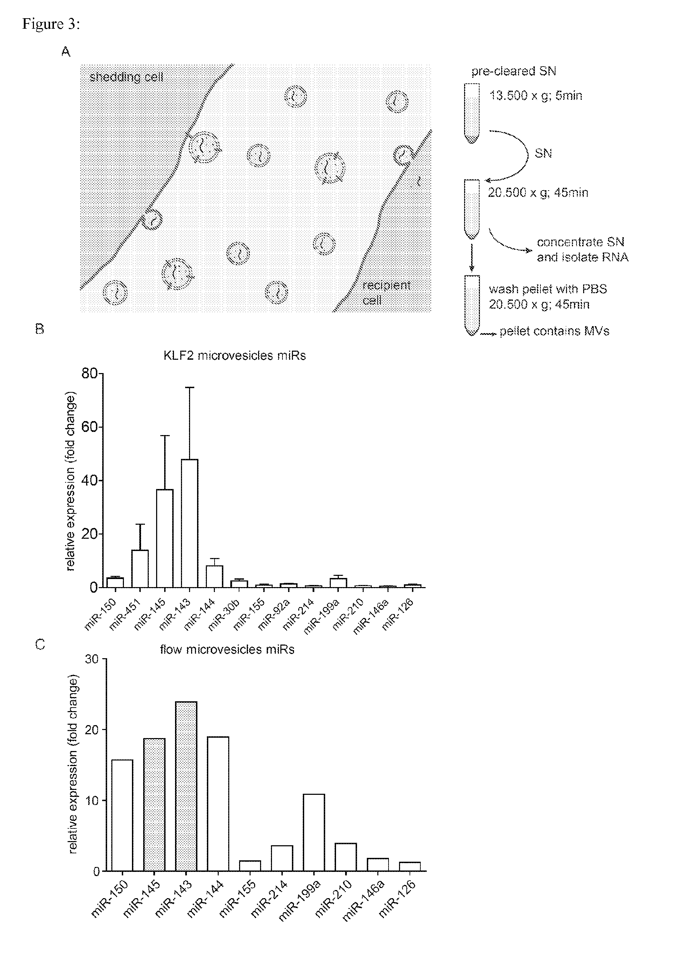

- **Figure 3**: shows KLF2 and shear stress induce miR-143 and miR-145 content of microvesicles. Microvesicles are generated by an active cellular process and can be isolated from cell supernatant in vitro or for example blood in vivo by ultracentrifugation (A). Microvesicles were isolated by ultracentrifugation as described in (A) from supernatant of mock- and KLF2- transduced HUVECs (B) or from HUVECs exposed to shear stress for 3 days and static controls (C). MicroRNAs were measured in RNA isolated from these microvesicles.

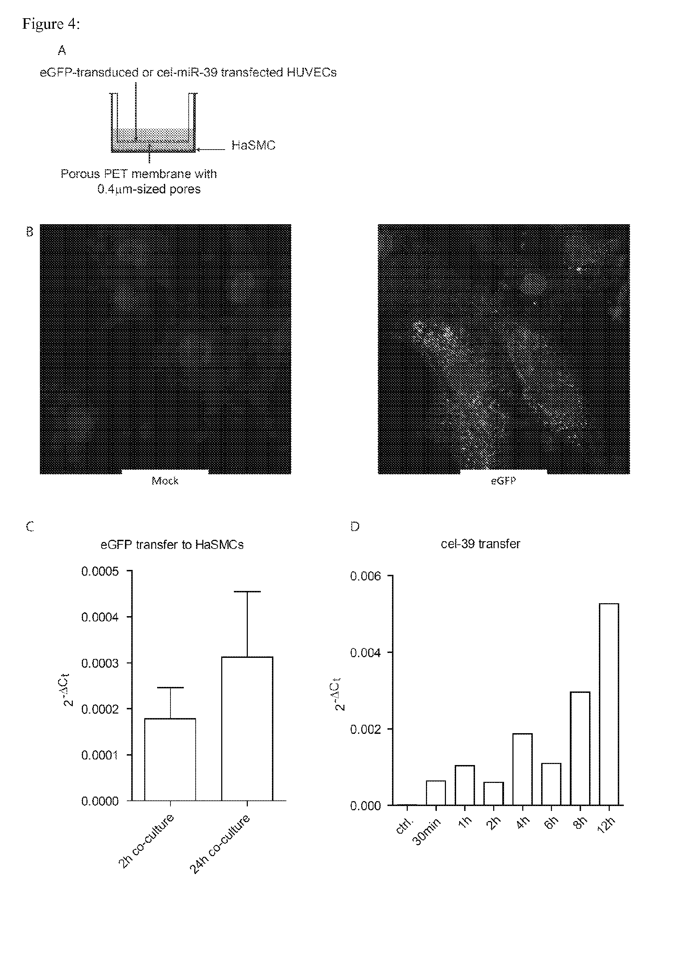

- **Figure 4**: shows endothelial cells transmit RNAs to smooth muscle cells in vitro. An in vitro co-culture system was used where endothelial cells are seeded in the top compartment, which is separated by a porous membrane from SMCs that are cultured in the bottom compartment (A). HUVECs (top compartment) were transduced with mock- or eGFP overexpression lentivirus and co-cultured with SMCs (bottom compartment). eGFP expression in SMCs was analyzed by confocal microscopy 72h after initiation of co-culture (B) or RNA was isolated and eGFP mRNA levels were measured by real-time PCR at 2h and 24h after start of co-culture (C). HUVECs were transfected with cel-miR-39 or left untransfected and RNA was isolated from SMCs at different time points after start of the coculture (D). Cel-miR-39 levels in SMCs were measured by real-time PCR.

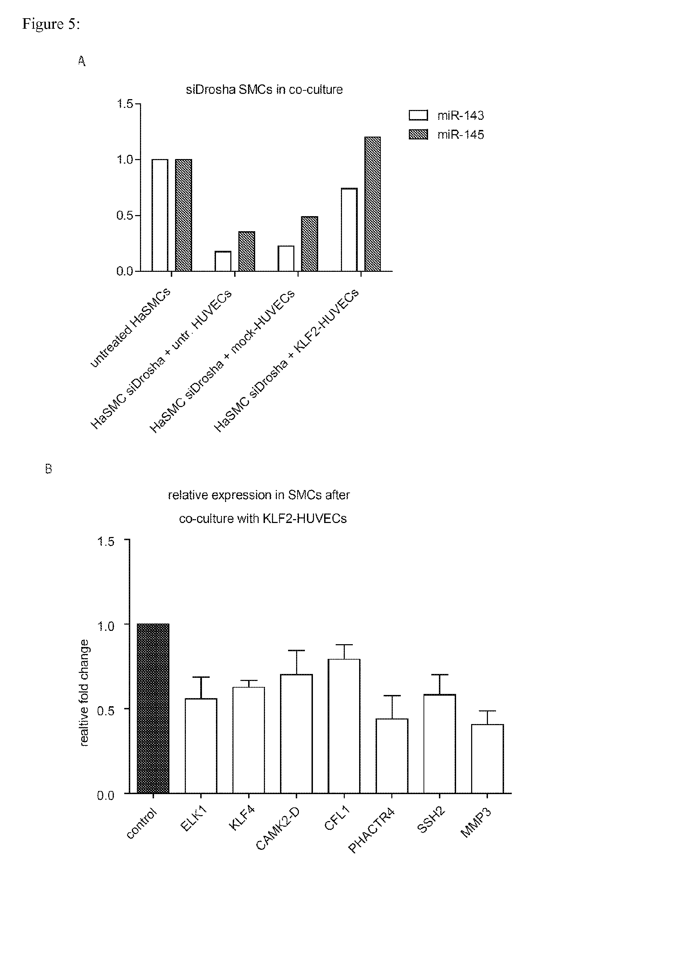

- **Figure 5**: shows endothelial cells transmit functional miR-143 and miR-145 to smooth muscle cells in vitro. HUVECs were transduced with mock- or KLF2-overexpression lentivirus or left untreated and co-cultured with SMCs that were transfected with an siRNA to silence Drosha expression or left untreated (A). RNA was isolated from the SMCs at 24h after the start of co-culture and miR-143 (white bars) and miR-145 (grey bars) expression in SMCs was analyzed by real-time PCR. Mock- (black bar) or KLF2-transduced (white bars) HUVECs were co-cultured with untreated SMCs for 24h and the expression levels of ELK1 (miR-143 target), KLF4 and CAMK2d (miR-145 targets), CFL1, PHACTR4, and SSH2 (targets of both miR-143 and miR-145) and MMP3 were analyzed by real-time PCR (B).

### Examples

### Material and Methods

### Mice

LacZ-reporter mice with miR-143/-145 deletion by insertion of an IRES-LacZ-NeoR cassette were generated by Boettger et al. (11) and generously provided to us for LacZ expression analysis.

### Cell culture

Human umbilical vein endothelial cells (HUVECs) were purchased from Lonza and cultured in EBM (Lonza) supplemented with 10% FBS and EGM-Singlequots (Lonza). Confluent monolayers were grown and used for the experiments. Human aortic smooth muscle cells (HaSMCs) were purchased from Lonza and cultured in SmBM (Lonza) supplemented with 5% FBS and SmGM-2-Singlequots (Lonza). HaSMCs were splitted after reaching 80% confluency to prevent cell-senescence.

### Shear stress

Twenty-four hours after the initial plating, confluent HUVEC monolayers were exposed to a well-defined laminar flow with a shear stress of 20 Dynes/cm2 for 72h in µ-Slides I0.4 Luer (ibidi) maintained and controlled by an Ibidi perfusion system. Control cells were seeded into µ-Slides I with reservoirs of culture-medium for long term cultivation without flow.

### Lentiviral particle generation and transduction

Long-term lentiviral overexpression of KLF2 was performed as previously described (16) Real-time PCR analysis TaqMan miRNA assays (Applied Biosystems) were used to measure the levels of mature miRNAs on a StepOne-Plus machine (Applied Biosystems). The snRNA U6 was used as a normalization control in all miRNA real-time experiments. The mRNA levels were determined using real-time analysis with SYBR (Applied Biosystems). The ribosomal protein P0 was used as a normalization control in all mRNA realtime experiments.

### Microvesicles isolation

Microvesicles were isolated using a multi-step centrifugation procedure as described previously (27): Briefly, confluent monolayers of HUVECs were grown and the medium was refreshed. After cultivation for 72h, the supernatant was collected and pre-cleared by centrifugation at 800g for 5 min. at 4°C. Next, the supernatant was centrifuged at 13.500g for 5 min. at 4°C to remove remaining cell debris. To pellet the microvesicles (size, < 1µm), the supernatant was centrifuged at 20.500g for 1h at 4°C. The supernatant was removed and discarded, while the pelleted microvesicles were washed with ice-cold PBS and pelleted again by centrifugation at 20.500g for 1h at 4°C. Finally, the supernatant was removed and discarded and the pelleted microvesicles were resuspended in 700µl Qiazol (Qiagen) for RNA isolation using the miRNeasy kit (Qiagen) following the instructions as provided by the manufacturer.

### Confocal microscopy

Confocal microscopy was done on a Zeiss laser scanning microscope LSM-510 (Zeiss, Germany) using Plan-Neofluar objectives (x20/0.5 or x40/1.3 Oil). Co-Culture experiments Well inserts for 6-well plates with a 0.4um pore-sized filter were purchased from Greiner and used following the manufacturer's instructions. 200.000 HUVECs were seeded into the well inserts and cultured in complete EBM 24 hours before the co-culture experiments. 100.000 HaSMCs were seeded into 6-well plates and cultured in complete SmBM 24 hours before the co-culture experiment. Before starting the co-culture experiments both, the HUVECs and the HaSMCs were washed with PBS. All co-culture experiments were done in complete EBM. In case of siDrosha transfected HaSMCs, the cells were transfected with a final concentration of 60nM siRNA targeting Drosha the day before the co-culture experiment using Lipofectamine RNAimax (Invitrogen) according to manufacturer's instructions.

### Example 1:

To assess which microRNAs are regulated by KLF2 in endothelial cells, the inventors employed an established lentiviral KLF2 overexpression system. HUVECs were transduced with a KLF2 lentivirus or mock control lentivirus and RNA was isolated after seven days. The inventors first measured the expression of a number of miRNAs with an established role in vascular biology by real-time PCR (fig 1A). Since KLF2 overexpression in the absence of shear stress does not fully mimic the effects of shear stress on endothelial cells (REF Boon, Horrevoets, Haemostaseologie 2010), the inventors further validated the expression of these miRNAs in endothelial cells that were exposed to shear stress for three days or kept under static conditions (fig 1C). Of note, KLF2 levels after lentiviral overexpression are similar to shear stress-induced expression of KLF2 (fig 1B and 1D).

Of all the miRNAs measured, miR-143 and miR-145, which are expressed in bicistronic cluster, are most robustly upregulated by KLF2 and shear stress stimulation. Until now, miR-143/145 expression has only been described in muscle cells and therefore the inventors analyzed the expression of miR-143/145 in vivo using LacZreporter mice, which showed that endothelial cells express miR-143/145 in vivo (fig 1E). These results show that miR-143/145 are expressed in endothelial cells of adult mice under physiological conditions.

### Example 2:

Having demonstrated that miR-143/145 expression is induced by KLF2 and shear stress, the inventors next analyzed whether the shear stress-mediated induction of miR-143/145 is dependent on KLF2 expression. To this end, the inventors used a lentiviral shRNA construct to silence KLF2 or a control shRNA in combination with shear stress stimulation in HUVECs. Flow stimulation was started four days after lentiviral transduction and RNA was isolated after three days of shear stress stimulation.

As is shown in figure 2A, shear stress stimulation in control-transduced cells leads to a similar induction of miR-143 and miR-145 as in untransduced HUVECs (fig 1B). Silencing of KLF2 impairs the shear stress-mediated induction of miR-143/145 to a similar extent as eNOS, an established direct transcriptional target of KLF2. Moreover, the best-described pharmacological inducers of KLF2, the anti-atherosclerotic HMG-CoA reductase inhibitors (statins), also induce the expression of miR-143/145 (fig 2B), further corroborating the regulation of this miRNA cluster by KLF2 in endothelial cells.

To investigate whether KLF2 directly induces the transcription of the miR-143/145 cluster, the inventors performed an *in silico* analysis of the promoter of miR-143/145 using rVIS-TA. The inventors identified one putative KLF-binding site in an evolutionary conserved part ∼6kb upstream of the miR-143/145 cluster (fig 2C). Next, the inventors transduced HUVECs with lentivirus encoding a KLF2-V5 fusion construct or with mock lentivirus and performed chromatin immunoprecipitation (ChIP) using V5 antibodies or non-specific IgG. Then the inventors analyzed by real-time PCR the amount of co-immunoprecipitated DNA using primers specific for miR-143/145 promoter region containing the putative KLF binding site (fig 2D) and found that KLF2 directly binds to the miR-143/145 promoter. Together, these results indicate that shear stress and statins induce miR-143/145 expression through direct transcriptional regulation by KLF2.

### Example 3:

KLF2 is thought to be the central mediator of an atheroprotective endothelial phenotype. It has recently been shown that atheroprotection can also be mediated by endothelial-derived apoptotic bodies. To study the regulation of microvesicle content by KLF2, the inventors isolated microvesicles by ultracentrifugation from the supernatants of mock- and KLF2-transduced HUVECs (fig 3A).

Next, RNA was isolated from microvesicles and the remaining supernatant and microRNA expression was measured by real-time PCR. KLF2 transduction resulted in a ∼20-fold enrichment of miR-143/145 in microvesicles (fig 3B), while KLF2 overexpression induces cellular miR-143/145 ∼10-fold, indicating a selective enrichment of miR-143/145 in microparticles induced by KLF2.

Next, the inventors tested whether shear stress exposure, as the physiological stimulus for KLF2 expression, alters the microRNA composition of microvesicles (fig 3C). Indeed, miR-143/145 expression was also highly enriched in microvesicles isolated from HUVECs that were exposed to shear stress, as compared to static controls. A number of other microRNAs was also significantly regulated, thus, microvesicles that are produced by KLF2-overexpressing or flow-exposed HUVECs have a unique microRNA composition.

### Example 4:

As the endothelial-specific KLF2-/- mouse does not exhibit any apparent endothelial cell defects, but does exhibit embryonic lethality as a result of a lack of SMCs organization around blood vessels, the inventors investigated whether endothelial-derived microvesicles can be transmitted to SMCs. To this end, a co-culture system was employed of HUVECs with human aortic SMCs in which the cells are separated by a 0.4*µ*m pore-sized membrane to prevent direct cell-to-cell communication or contact (fig 4A).

Firstly, HUVECs were used that were transduced with eGFP-overexpressing lentivirus or mock control lentivirus and analyzed the eGFP levels in SMCs after 3 days by confocal microscopy (fig 4B). This showed that eGFP can indeed be transferred from the endothelial cells to SMCs during the co-culture. Secondly, to delineate whether transfer of eGFP occurs in the form of protein or of mRNA encoding eGFP, the inventors isolated RNA from the SMCs and performed real-time PCR with primers specific for eGFP. Indeed, the inventors were able to measure eGFP mRNA in SMCs in a time-dependent manner (fig 4C).

Thirdly, HUVECs were transfected with a miRNA that is naturally only present in *C*. *Elegans* (cel-miR-39) and analyzed cel-miR-39 expression levels in SMCs in time after the start of the co-culture experiment (fig 4D). This showed that mRNAs and miRNAs can also be transferred from endothelial cells to SMCs. Taken together, these experiments demonstrate that endothelial cells can transfer cellular components to SMCs mediated by vesicles.

### Example 5:

SMCs normally contain high levels of miR-143/145, but these can be rapidly diminished by pro-atherogenic stimuli. To mimic this situation *in vitro,* the inventors transfected SMCs with a siRNA directed against Drosha, a critical component of the miRNA biogenesis pathway, and used these SMCs in co-culture experiments with endothelial cells (fig 5A).

Drosha knockdown leads to a ∼5-fold decrease in miR-143/145 levels in SMCs, which is similar to the atherosclerosis-mediated decrease in vivo. Co-culture with mock-transduced endothelial cells does not affect the levels of miR-143/145 in SMCs, while co-culture with KLF2- transduced endothelial cells dramatically induces miR-143/145 levels almost to the levels as measured without Drosha knockdown (fig 5A).

Then, the co-culture system was employed to study the effects of endothelial KLF2 on the expression ofmiR-143/145-regulated genes (fig 5B). Mock- and KLF2-transduced HUVECs were co-cultured with untreated SMCs for 24h in full EBM medium. RNA was subsequently isolated and the expression of validated miR-143 and miR-145 targets, as well as matrix metallo-proteinase 3 (MMP3) that has been associated with SMC de-differentiation, was measured by real-time PCR. The presence of KLF2-transduced endothelial cells reduces the expression of these established miR-143/145 target genes in SMCs, as compared to co-culture with control endothelial cells. Together, these results illustrate that transfer of miR-143/145 from endothelial cells to SMCs is induced by endothelial KLF2 expression, which leads to an enhanced repression of miR-143/145 target genes and de-differentiation-associated gene expression.

## Claims

1. A method for the production of atheroprotective vesicles, comprising the steps of
a. providing endothelial cells,
b. inducing and/or enhancing the activity of the Krüppel-like factor 2 (Klf2) in said endothelial cells,
c. incubating said endothelial cells for a time sufficient to allow the cells to produce vesicles, and
d. optionally, isolating said vesicles.

2. The method of claim 1, wherein said endothelial cells are mammalian cells, preferably human cells, in particular human vascular cells derived from an adult blood vessel, microvascular endothelial cells or embryonic umbilical vein cells (HUVECs).

3. The method according to claim 1 or 2, wherein inducing and/or enhancing the activity of Klf2 is achieved by
a) the further method steps comprising of
i. introducing into the endothelial cells a Klf2 expression construct, and
i. expressing Klf2 in the endothelial cells; and/or
b) applying shear stress to said endothelial cells; and/or
c) treatment of said endothelial cells with one or more inducers of Klf2.

4. The method according to claim 3, wherein the Klf2 expression construct is a lentiviral expression construct.

5. The method according to claim 3 or 4, wherein shear stress is applied by flow stimulation of the endothelial cells, in particular by applying a laminar flow to the endothelial cells causing a shear stress of about >12 Dynes/cm².

6. The method according to any one of claims 3 to 5, wherein the inducer of Klf2 is HMG-CoA reductase inhibitor, specifically selected from the group of the statins consisting of Atorvastatin, Cerivastatin, Fluvastatin, Lovastatin, Mevastatin, Pitavastatin, Pravastatin, Rosuvastatin and Simvastatin, or combinations thereof, preferably wherein the statin is Mevastatin.

7. The method according to any one of claims 1 to 6, wherein the vesicles are isolated from the endothelial cell culture supernatant and/or from the endothelial cell suspension, preferably by multi-step centrifugation.

8. A vesicle produced with a method according to any one of claims 1 to 7.

9. A vesicle, enclosing at least one micro RNA selected from the group consisting of miR-150, miR-451, miR-145, miR-143, miR-144, miR-193a-3p, miR-155, miR-181a, miR-214, miR-199b, miR-199a, miR-210, miR-146a, miR-126, miR-378, miR-363 and miR-30b,

10. The vesicle according to claim 8 or 9, the vesicle enclosing at least one, preferably all, of the miRNAs miR-145, miR-143, miR-150, miR-144, miR-199a and/or miR-451.

11. The vesicle according to any one of claims 8 to 10, wherein at least one, preferably both, of the miRNAs miR-126 and miR-210 are not present, or present in low concentrations or similar concentrations compared to vesicles derived from endothelial cells wherein KLF2 was not enhanced and/or induced.

12. A vesicle according to any one of claims 8 to 11, for use in the treatment or prevention of a vascular disease, wherein the vascular disease is selected from the group consisting of acute coronary syndromes, such as myocardial infarction, unstable angina or sudden cardiac death, plaque rupture, aneurisms, in-stent restenosis and ischemic diseases, such as ischemic stroke, peripheral arterial occlusive disease, acute and chronic ischemic cardiac disease, all types of heart failure, hibernating myocardium and microcirculory dysfunction.

13. A pharmaceutical composition, comprising a vesicle according to any one of claims 8 to 12 together with a pharmaceutical acceptable carrier and/or exipient.

14. A method of modulating a smooth muscle cell, comprising the steps of
a. providing a smooth muscle cell,
b. treating the smooth muscle cell with vesicles according to any one of claims 8 to 11, or
c. co-culturing said smooth muscle cell with endothelial cells wherein the activity of Klf2 is induced and/or enhanced.

15. A method according to claim 19, wherein the smooth muscle cell is preferably a mammalian cell, most preferably a human cell, such as a vascular smooth muscle cell, for example a human aortic smooth muscle cell.

16. A method according to claim 14 or 15, wherein the smooth muscle cell is provided in the context of a tissue, such as a blood vessel, preferably wherein the tissue is an explanted tissue or an artificial tissue.
